# EUROPÄISCHE PATENTSCHRIFT

(11) **EP 2 962 617 B1**
(45) Veröffentlichungstag und Bekanntmachung des Hinweises auf die Patenterteilung: **06.10.2021**
(21) Anmeldenummer: 15173822.6
(22) Anmeldetag: 25.06.2015
(51) Int. Cl.: A47L 15/50, A61L 2/24, B08B 3/04, B62B 3/00, A47L 15/00

(54) **TRANSPORTWAGEN FÜR SPÜLKÖRBE EINES REINIGUNGS- UND DESINFEKTIONSGERÄTS**
TRANSPORT TROLLEY FOR WASHING BASKETS OF A CLEANING AND DISINFECTING DEVICE
CHARIOT DE TRANSPORT POUR CASIER DE RINÇAGE D'UN DISPOSITIF DE DESINFECTION ET DE NETTOYAGE

(30) Priorität: 02.07.2014 DE 102014109239
(43) Veröffentlichungstag der Anmeldung: 06.01.2016
(73) Patentinhaber: Miele & Cie. KG, 33332 Gütersloh (DE)
(72) Erfinder: Malec, Tobias, 33739 Bielefeld (DE)

(56) Entgegenhaltungen:
- DE-A1- 1 628 535
- DE-A1- 19 513 994
- DE-A1- 19 912 564
- GB-A- 963 537
- US-A- 5 904 162
- US-A1- 2003 030 239

## Beschreibung

Die Erfindung betrifft einen Transportwagen für Spülkörper eines Reinigungs- und Desinfektionsgerätes, mit einem Traggestell, das zur Aufnahme eines Spülkorbs zwei voneinander beabstandet angeordnete Tragschienen aufweist.

Reinigungs- und Desinfektionsgeräte kommen insbesondere im gewerblichen Bereich zum Einsatz. Sie verfügen in an sich bekannter Weise über einen Spülbehälter, der einen Spülraum bereitstellt. Der Spülraum ist über eine Beschickungsöffnung zugänglich, die im bestimmungsgemäßen Verwendungsfall mittels einer Spülraumtür fluiddicht verschließbar ist.

Zur Beschickung eines Reinigungs- und Desinfektionsgerätes mit zu reinigendem und/oder zu desinfizierendem Spülgut kommen Spülkörbe zum Einsatz, die dem Spülraum des Reinigungs- und Desinfektionsgerätes entnehmbar sind, so dass eine Bestückung eines Spülkorbs mit Spülgut außerhalb des Reinigungs- und Desinfektionsgerätes stattfinden kann. Nach einer Bestückung eines Spülkorbs mit zu reinigendem Spülgut kann der Spülkorb zusammen mit dem zu reinigenden Spülgut durch die Beschickungsöffnung in den Spülraum des Reinigungs- und Desinfektionsgerätes verbracht werden, woraufhin dann eine bestimmungsgemäße Reinigung- und/oder Desinfektion des Spülgutes vorgenommen werden kann.

Zur Handhabung eines Spülkorbs außerhalb des Spülraums eines Reinigungs- und Desinfektionsgerätes bzw. zur Spülkorbbe- und/oder entladung kommen sogenannte Transport- oder Transferwagen zum Einsatz. Diese verfügen über ein mit Rollen bestücktes Traggestell, das für eine bestimmungsgemäße Aufnahme eines Spülkorbs zwei voneinander beabstandet angeordnete Tragschienen aufweist. Diese Tragschienen stützen im bestimmungsgemäßen Verwendungsfall einen vom Transportwagen aufgenommenen Spülkorb ab und können zum Zwecke der vereinfachten Handhabung spülkorbseitig über Laufrollen verfügen, was ein vereinfachtes Einschieben eines von einem Traggestell aufgenommenen Spülkorbs in den Spülraum eines Reinigungs- und Desinfektionsgerätes ermöglicht.

Die aus dem Stand der Technik vorbekannten Transportwagen haben sich im alltäglichen Praxiseinsatz bewährt. Sie sind gleichwohl nicht frei von Nachteilen.

So können ordnungsgemäß bestückte Spülkörbe je nach aufgenommenem Spülgut 200 kg und mehr wiegen. Dementsprechend kann sich das Einschieben eines Spülkorbs vom Transportwagen in den Spülraum eines Reinigungs- und Desinfektionsgerätes als schwergängig erweisen. Die benutzerseitig aufzubringende Kraft kann hierbei zu Beginn der Verschiebung des Spülkorbs noch relativ gut auf den Spülkorb übertragen werden. Mit zunehmender Entfernung zwischen Spülkorb einerseits und Verwender andererseits wird dies aber zunehmend schwieriger, insbesondere bei kleineren Personen. Je nach Armlänge des Verwenders kann es sogar sein, dass der Verwender neben den Transportwagen treten muss, um den davon getragenen Spülkorb auch noch über die letzte Wegstrecke in den Spülraum des Reinigungs- und Desinfektionsgerätes verbringen zu können. Das Verschieben des Spülkorbs erfordert dann noch mehr Kraftaufwand, da die Kraftübertragung in den zu verschiebenden Spülkorb seitlich erfolgt. Zudem kann es infolge des seitlichen Spülkorbangriffs zu einer Verkantung des Spülkorbs an den Tragschienen des Transportwagens kommen. Der Verwender hat dann in diesem Fall auf die gegenüberliegende Seite des Transportwagens zu wechseln, um über eine Krafteinleitung über die andere Seite des Spülkorbs eine Entkantung zu erwirken. Dies erfordert nicht nur einen erhöhten Kraftaufwand, es ist auch umständlich und wird insofern verwenderseitig als nachteilig empfunden.

Die US 5904162 A offenbart einen Transportwagen für eine gewerbliche Spülmaschine mit einer Aufnahmeschale zur Aufnahme eines Spülkorbes, die fest an einer Tragestruktur angeordnet ist. Die Tragestruktur weist lediglich an ihrer im Beschickungsfall von der Spülmaschine abgewandten Seite Beine auf, wodurch in Verbindung mit der Spülmaschine eine platzsparende Anordnung des Transportwagens im Nichtgebrauchsfall ermöglicht ist.

Weiterhin offenbart auch die DE 1 628 535 A1 einen Transportwagen für Spülkörbe eines Reinigungs-und Desinfektionsgerätes, mit einem Traggestell, das zur Aufnahme eines Spülkorbs voneinander beabstandet angeordnete Tragschienen aufweist.

Die **Aufgabe** der Erfindung ist es, einen Transportwagen bereitzustellen, der verwenderseitig eine vereinfachte Handhabung ermöglicht.

Zur **Lösung** dieser Aufgabe wird mit der Erfindung ein Transportwagen mit den Merkmalen von Anspruch 1 vorgeschlagen.

Der Transportwagen nach der Erfindung verfügt in an sich bekannter Weise über Tragschienen, die im bestimmungsgemäßen Verwendungsfall der Abstützung eines Spülkorbs dienen. Zum Zwecke der Beschickung eines Spülraums eines Reinigungs- und Desinfektionsgerätes ist der Transportwagen in Beschickungsrichtung vor dem Spülraum des Reinigungs- und Desinfektionsgerätes zu positionieren. In dieser Position verlaufen die Tragschienen in ihrer Längsrichtung in Richtung zur Beschickungsrichtung.

Die in dieser Position des Transportwagens dem Reinigungs- und Desinfektionsgerät abgewandte Seite des Traggestells des Transportwagens ist erfindungsgemäß offen ausgebildet. An dieser Seite des Traggestells sind die Tragschienen somit nicht miteinander verbunden, d.h. an dieser Seite des Traggestells befindet sich keine quer zu den Tragschienen erstreckende Verbindungsstrebe. Diese offene Ausgestaltung des Traggestells ermöglicht es einem Verwender, beim Einschieben eines vom Transportwagen aufgenommenen Spülkorbs in den Spülraum des Reinigungs- und Desinfektionsgerätes zwischen die Tragschienen des Transportwagens zu treten. Ein Verwender kann mithin beim Einschieben eines Spülkorbs in den Spülraum des Reinigungs- und Desinfektionsgerätes in Beschickungsrichtung auf das Reinigungs- und Desinfektionsgerät zugehen, da die in Beabstandungsrichtung einendseitig der Tragschienen verlaufende Traggestellseite offen ausgebildet ist. Dies vereinfacht im Unterschied zum Stand der Technik in vorteilhafter Weise die Spülkorbhandhabung, da ein Verwender für ein vollständiges Einschieben eines Spülkorbs in den Spülraum des Reinigungs- und Desinfektionsgerätes nicht mehr neben den Transportwagen zu treten hat.

Das Traggestell verfügt gemäß einem weiteren Merkmal der Erfindung anderendseitig der Tragschienen über eine sich quer zu den Tragschienen erstreckende Verbindungsstrebe. Diese dient der Stabilisierung und der insgesamt robusten Ausgestaltung des Traggestells. Es können mehrere solcher Verbindungsstreben vorgesehen sein, wobei gemäß einem weiteren Merkmal der Erfindung vorgesehen ist, dass eine dieser Verbindungsstreben an die Tragschienen angeschlossen ist. Gemäß dieser bevorzugten Ausführungsform bilden die Tragschienen und die dazwischen angeordnete Verbindungsstrebe eine U-förmige Ausgestaltung aus. Dabei bilden die beiden Tragschienen die Schenkel dieser U-förmigen Ausgestaltung. Anderes ausgedrückt verfügt das Traggestell insbesondere ausschließlich anderendseitig der Tragschienen, also auf der in der Beschickungsposition dem Reinigungs- und Desinfektionsgerät zugewandte Seite des Traggestells des Transportwagens, über eine sich quer zu den Tragschienen erstreckende Verbindungsstrebe. Somit kann der Verwender beim Einschieben eines vom Transportwagen aufgenommenen Spülkorbs in den Spülraum des Reinigungs- und Desinfektionsgerätes zwischen den Tragschienen des Transportwagens bis unmittelbar vor das Reinigungs- und Desinfektionsgerät auf das Reinigungs- und Desinfektionsgerät zugehen.

Die Tragschienen verfügen gemäß einem weiteren Merkmal der Erfindung jeweils über eine Mehrzahl von ersten Laufrollen. Diese sind in Richtung der Tragschienen hintereinander angeordnet und dienen als Korblaufrollen der Aufnahme des Spülkorbs. Im bestimmungsgemäßen Anwendungsfall wird der Spülkorb beim Einschieben in den Spülraum des Reinigungs- und Desinfektionsgerätes von den Korblaufrollen getragen, womit der Einschiebwiderstand auf ein Minimum reduziert ist.

Die Tragschienen verfügen gemäß einem weiteren Merkmal der Erfindung jeweils über eine Mehrzahl von zweiten Laufrollen, die in Längsrichtung der Tragschienen jeweils hintereinander angeordnet sind. Diese zweiten Laufrollen dienen der Abstützung einer Auffangwanne. Dabei ist es Sinn und Zweck dieser Auffangwanne vom noch nicht gereinigten Spülgut abtropfende Flüssigkeiten aufzufangen, um eine Verschmutzung des Fußbodens und des Transportwagens insbesondere des Traggestells zu vermeiden.

Die ersten Laufrollen überragen gemäß einem weiteren Merkmal der Erfindung die zweiten Laufrollen in Höhenrichtung. Diese Ausgestaltung gestattet es in vorteilhafterweise, Spülkorb und Wanne unabhängig voneinander verschieben zu können.

Gemäß einer bevorzugten Ausführungsform der Erfindung ist vorgesehen, dass die Wanne einendseitig einen Spülkorbmitnehmer aufweist. Diese Ausgestaltung erweist sich insbesondere aus handhabungstechnischen Gründen als vorteilhaft, da sie eine Vereinfachung erbringt. So lässt sich verwenderseitig die Wanne ergreifen und in Beschickungsrichtung, das heißt in Richtung auf den vom Reinigungs- und Desinfektionsgerät bereitgestellten Spülraum verschieben. Der an der Wanne angeordnete Korbmitnehmer nimmt infolge dieser Wannenverschiebung den Spülkorb mit, der sodann ebenfalls in Richtung auf den Spülraum des Reinigungs- und Desinfektionsgerätes verschoben wird. Dabei rollen infolge dieser Verschiebebewegung die Wanne auf den zweiten Laufrollen und der Spülkorb auf den ersten Laufrollen des Transportwagens ab. Sobald der Spülkorb bestimmungsgemäß in den Spülraum des Reinigungs- und Desinfektionsgerätes verbracht ist, kann die Auffangwanne zurückverfahren und in ihre Ausgangsposition am Transportwagen verbracht werden. Dabei verleibt der Spülkorb im Spülraum.

Das Mitfahren der Wanne bei einem Einschieben des Spülkorbs in den Spülraum des Reinigungs- und Desinfektionsgerätes erbringt im Unterschied zum Stand der Technik den zusätzlichen positiven Effekt, dass etwaige vom noch zu reinigendem Spülgut abtropfende Restflüssigkeiten nicht in den Spaltraum zwischen Transportwagen und Reinigungs- und Desinfektionsgerät gelangen können. Da im Übrigen im Unterschied zum Stand der Technik die Wanne nicht am Transportwagen festmontiert ist, kann sie zum Zwecke einer Reinigung entnommen oder alternativ durch eine schon vorgereinigte Wanne ersetzt werden. Auch insoweit ist die Handhabung hinsichtlich des erfindungsgemäßen Transportwagens vereinfacht.

Gemäß einem weiteren Merkmal der Erfindung ist vorgesehen, dass das Traggestell auf seine der offenen Seite gegenüberliegenden Seite einen mit dem Reinigungs- und Desinfektionsgerät zusammenwirkenden Verriegelungsmechanismus aufweist. Mittels dieses Verriegelungsmechanismusses kann der Transportwagen mechanisch mit dem Reinigungs- und Desinfektionsgerät gekoppelt werden, womit eine Spülkorbbe- und entladung des Reinigungs- und Desinfektionsgerätes möglich ist, ohne dass die Gefahr besteht, dass sich der Transportwagen bei einer Verschiebebewegung des Spülkorbs ungewollt vom Reinigungs- und Desinfektionsgerät entfernt.

Weitere Merkmale und Vorteile der Erfindung ergeben sich aus der nachfolgenden Beschreibung anhand der Figuren. Dabei zeigen:
- Figur 1: in schematisch perspektivischer Ansicht ein Reinigungs- und Desinfektionsgerät mit einem erfindungsgemäßen Transportwagen;
- Figur 2: die Darstellung nach Figur 1 ohne Spülkorb;
- Figur 3: in einer Detailansicht ausschnittsweise den erfindungsgemäßen Transportwagen in perspektivischer Ansicht;
- Figur 4: in einer Detailansicht ausschnittsweise den erfindungsgemäßen Transportwagen in frontseitiger Ansicht;
- Figur 5: in einer Detailansicht den Verriegelungsmechanismus des erfindungsgemäßen Transportwagens und
- Figur 6: ein Reinigungs- und Desinfektionsgerät mit einem Transportwagen nach dem Stand der Technik.

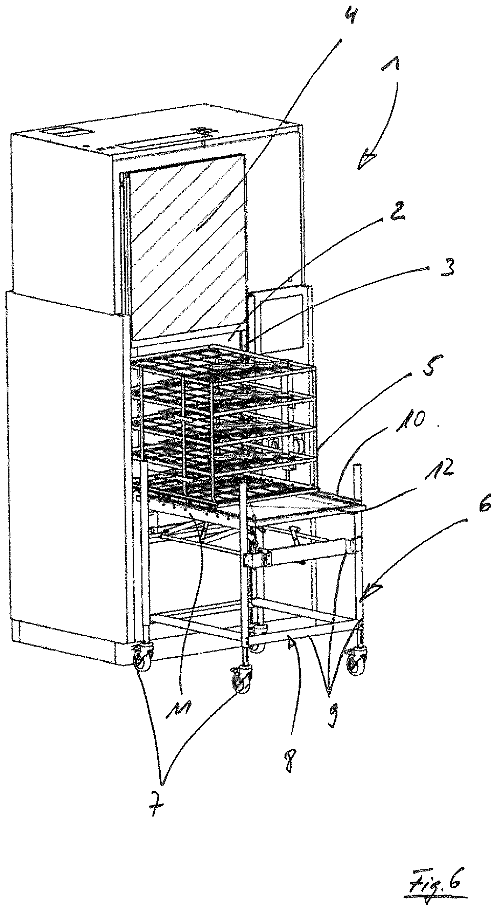

Figur 6 lässt in schematisch perspektivischer Darstellung ein Reinigungs- und Desinfektionsgerät mit einem Transportwagen 6 nach dem Stand der Technik erkennen.

Das Reinigungs- und Desinfektionsgerät verfügt in an sich bekannter Weise über einen Spülraum 2, der über eine Beschickungsöffnung 3 zugänglich ist. Die Beschickungsöffnung 3 ist mittels einer Spülraumtür fluiddicht verschließbar.

Zur Beschickung des Spülraums 2 mit zu reinigendem Spülgut dient ein Spülkorb 5. Dieser ist dem Spülraum 2 entnehmbar und außerhalb des Spülraums 2 mit Spülgut zu bestücken. Für eine Handhabung des Spülkorbs 5 außerhalb des Spülraums 2 bzw. für eine Spülkorbbe- und entladung des Reinigungs- und Desinfektionsgerätes 1 dient ein Transportwagen 6. Figur 6 lässt einen Transportwagen 6 nach dem Stand der Technik erkennen.

Der aus dem Stand der Technik vorbekannte Transportwagen 6 verfügt über ein Traggestell 8, das eine Mehrzahl von Streben 9 aufweist. Fußbodenseitig ist das Traggestell 8 mit Rollen 7 bestückt. Das Traggestell 8 stellt ferner zwei Tragschienen 10 und 11 bereit, deren Längserstreckung bei einer Spülkorbbe- bzw. entladung in Beschickungsrichtung 14 verläuft. Zwischen den beiden Tragschienen 10 und 11 ist eine am Traggestell 8 angeordnete Wanne 12 vorgesehen, die dazu dient, etwaige Restflüssigkeiten aufzufangen, die von noch nicht gereinigtem Spülgut abtropfen können. Wie sich aus der Darstellung nach Figur 6 ergibt, weist der Transportwagen 6 eine von oben betrachtet insgesamt rechteckigförmige Ausgestaltung auf. Für eine ordnungsgemäße Beschickung des Spülraums 2 ist es verwenderseitig erforderlich, neben den Transportwagen 2 zu treten, um den Spülkorb 5 vollends in den vom Reinigungs- und Desinfektionsgerät 1 bereitgestellte Spülraum 2 einzufahren.

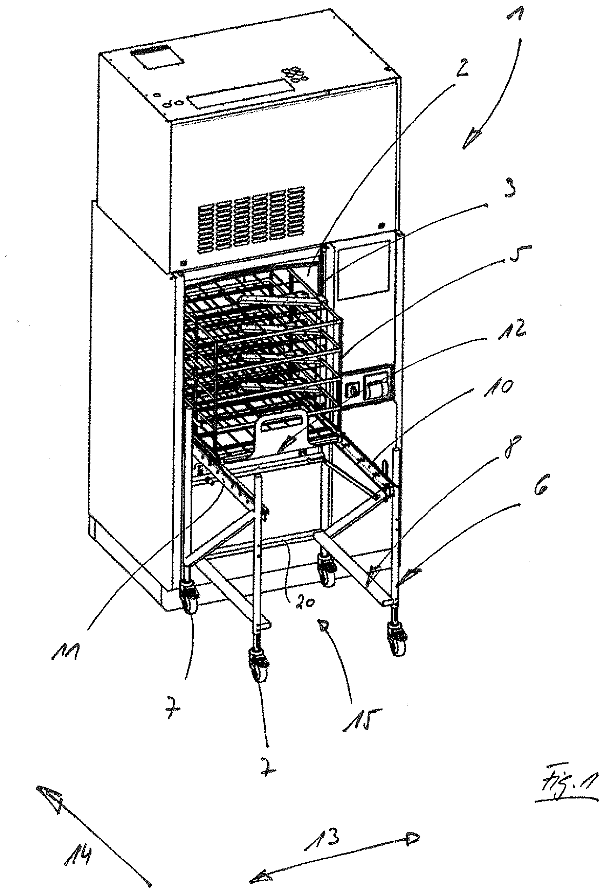

Figur 1 zeigt einen Transportwagen 6 nach der Erfindung. Dieser verfügt ebenfalls über ein Traggestell 8, das zur Aufnahme eines Spülkorbs 5 zwei voneinander beabstandete Tragschienen 10 und 11 aufweist. Im Unterschied zum Stand der Technik ist aber vorgesehen, dass die in Beabstandungsrichtung 13 einendseitig der Tragschienen 10 und 11 verlaufende Traggestellseite 15 offen ausgebildet ist. Die offene Ausgestaltung der im Be- und Entladefall dem Reinigungs- und Desinfektionsgerät 1 gegenüberliegende Traggestellseite bringt den Vorteil, dass verwenderseitig bei einem Einschieben des Spülkorbs 5 in den Spülraum 2 bzw. bei einem Entladevorgang zwischen die beiden Tragschienen 10 und 11 getreten werden kann. Eine Handhabung des Spülkorbs 5 von einer Seite des Transportwagens 6 her ist nicht erforderlich. Damit wird eine wesentliche Vereinfachung bei der Handhabung erreicht. Zudem wird vermieden, dass es zu Verkantungen des Spülkorbs 5 relativ gegenüber dem Traggestell 8, insbesondere den Tragschienen 10 und 11 kommen kann. Verwenderseitig ist ein gleichmäßiges und gleichförmiges Einschieben durch zentrale Krafteinleitung möglich.

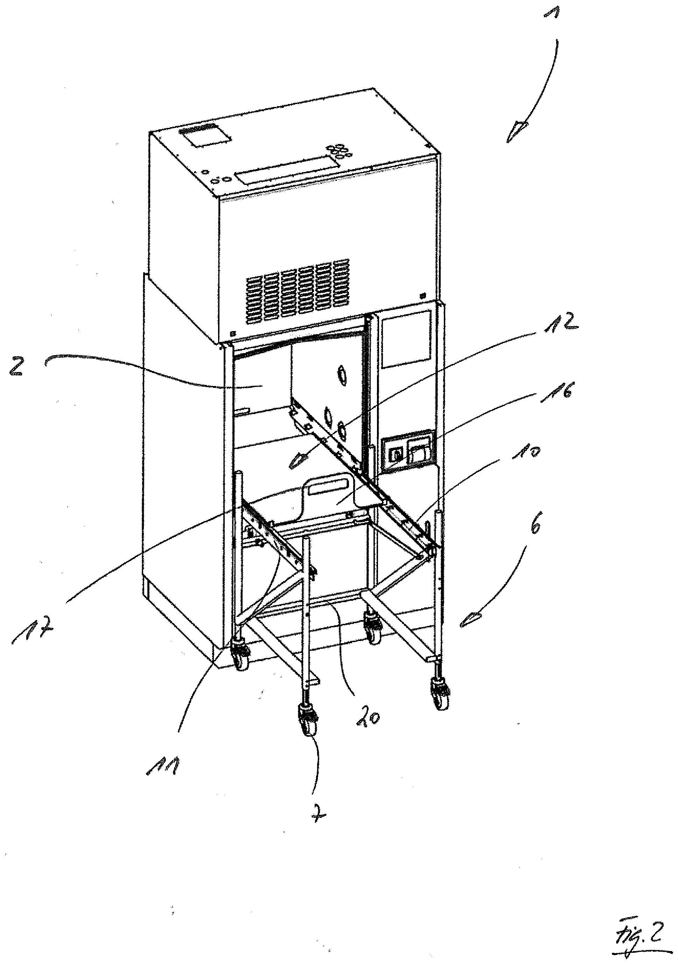

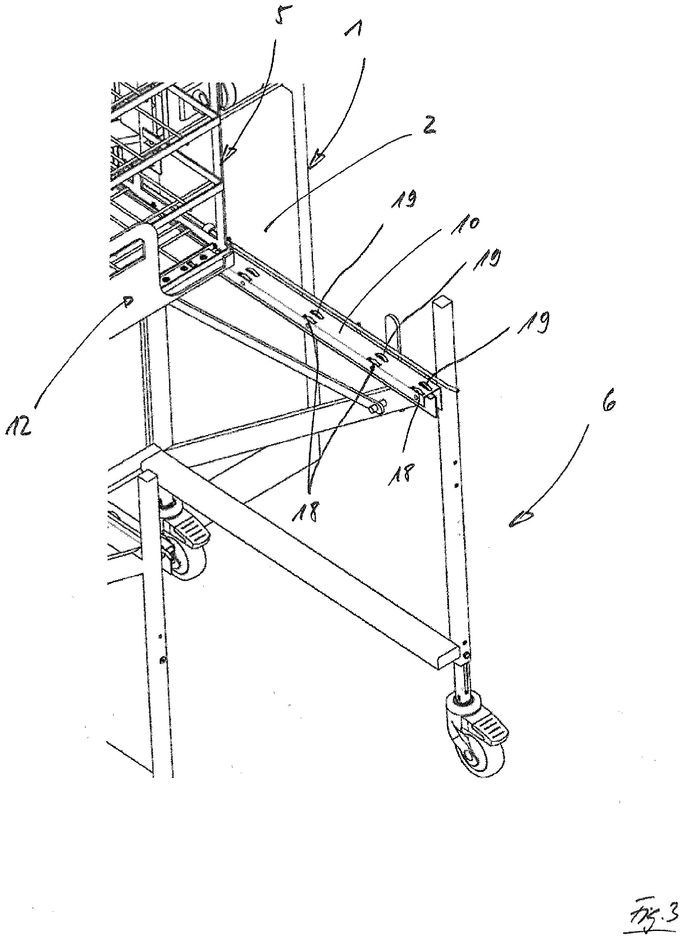

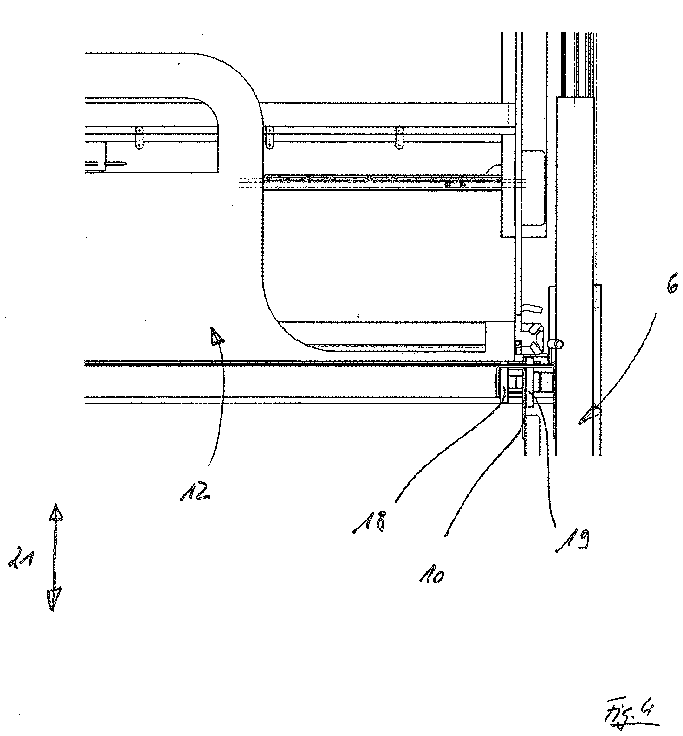

Wie eine Zusammenschau der Figuren 1 und 2 erkennen lässt, ist unterhalb des Spülkorbs 5 eine Wanne 12 angeordnet. Diese ist allerdings im Unterschied zum Stand der Technik nicht fest mit dem Traggestell 8 verbunden, sondern vielmehr ebenfalls wie der Spülkorb 5 in Beschickungsrichtung 14 verfahrbar vom Traggestell 8, das heißt den Tragschienen 10 und 11 aufgenommen. Zu diesem Zweck verfügen die Tragschienen 10 und 11 über Laufrollen, und zwar zum einen über Korblaufrollen 19 sowie zum anderen über Wannenlaufrollen 18, wie sich dies aus einer Zusammenschau der Figuren 3 und 4 ergibt.

Wie Figur 3 erkennen lässt, verfügt jede Tragschiene 10 bzw. 11 über eine Mehrzahl von als Korblaufrollen 19 dienende Rollen, die in Längsrichtung der Tragschienen 10 bzw. 11 hintereinander angeordnet sind. Auf diesen Korbrollen 19 stützt sich der Spülkorb 5 ab.

Jede Tragschiene 10 bzw. 11 verfügt desweiteren über eine Mehrzahl von zweiten Laufrollen, den sogenannten Wannenlaufrollen 18. Diese sind in Längsrichtung der Tragschienen 10 bzw. 11 ebenfalls hintereinander angeordnet und dienen der Abstützung der Wanne 12.

Wie sich aus der Darstellung nach Figur 4 ergibt, überragen die Korblaufrollen 19 die Wannenlaufrollen 18 in Höhenrichtung 21. Dies gestattet es, den Spülkorb 15 unabhängig von der Wanne 12 in Beschickungsrichtung 14 zu verfahren.

Die Wanne 12 verfügt über einen Korbmitnehmer 16. Dieser ist im gezeigten Ausführungsbeispiel als abgekantetes Blechteil der Wanne 12 ausgebildet. Der Mitnehmer 16 stellt zudem in Form eines Griffs eine Handhabe 17 bereit, die eine verwenderseitige Benutzung vereinfacht.

Im bestimmungsgemäßen Verwendungsfall kann ein Verwender die Wanne 12 an der Handhabe 17 ergreifen und diese in Beschickungsrichtung 14 in den Spülraum 2 des Reinigungs- und Desinfektionsgerätes 1 verfahren. Infolge dieser Verfahrbewegung wird der Spülkorb 5 von dem an der Wanne 12 angeordneten Mitnehmer 16 mitgenommen, das heißt ein Verschieben der Wanne 12 führt auch zu einem Verschieben des Spülkorbs 5 in Beschickungsrichtung 14. Dabei wird die Wanne von den Wannenlaufrollen 18 und der Spülkorb von den Korbrollen 19 getragen.

Sobald der Spülkorb 5 in bestimmungsgemäßer Weise in den Spülraum 2 des Reinigungs- und Desinfektionsgerätes 1 verbracht ist, kann die Wanne 12 entgegen der Beschickungsrichtung 14 zurück in ihre Ausgangsposition verfahren werden. Die mitverfahrende Wanne 12 hat insbesondere den Vorteil, dass etwaige Restflüssigkeiten, die vom noch nicht gereinigten Spülgut abtropfen können, nicht in den Spaltraum zwischen Reinigungs- und Desinfektionsgerät 1 und Transportwagen 6 gelangen können.

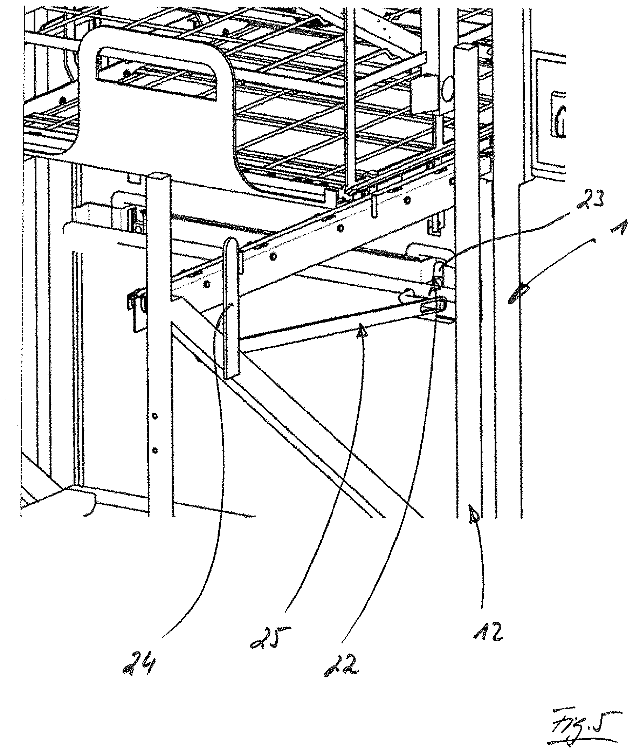

Wie die Detailansicht nach Figur 5 ferner erkennen lässt, verfügt der Transportwagen 6 über einen Verriegelungsmechanismus 22. Dieser stellt reinigungs- und desinfektionsgeräteseitig einen Bolzen 23 zur Verfügung, der eine mechanische Verrastung von Transportwagen 6 und Reinigungs- und Desinfektionsgerät 1 gestattet. Es kann so gefahrlos eine Spülkorbbe- und entladung stattfinden. Für eine Verriegelung des Bolzens 23 dient eine verwenderseitig zu ergreifende Handhabe 24, die über eine Gestängeanordnung 25 mit dem Bolzen 23 in Wirkverbindung steht.

### Bezugszeichen

- 1: Reinigungs- und Desinfektionsgerät
- 2: Spülraum
- 3: Beschickungsöffnung
- 4: Spülraumtür
- 5: Spülkorb
- 6: Transportwagen
- 7: Rolle
- 8: Traggestell
- 9: Strebe
- 10: Tragschiene
- 11: Tragschiene
- 12: Wanne
- 13: Beabstandungsrichtung
- 14: Beschickungsrichtung
- 15: Traggestellseite
- 16: Mitnehmer
- 17: Handhabe

- 18: Wannenlaufrolle
- 19: Korblaufrolle
- 20: Verbindungsstrebe
- 21: Höhenrichtung
- 22: Verriegelungsmechanismus
- 23: Bolzen
- 24: Handhabe
- 25: Gestängeanordnung

## Patentansprüche

1. Transportwagen für Spülkörbe eines Reinigungs- und Desinfektionsgerätes, mit einem Traggestell (8), das zur Aufnahme eines Spülkorbs (5) zwei voneinander beabstandet angeordnete Tragschienen (10, 11) aufweist, wobei der Transportwagen zum Zwecke der Beschickung eines Spülraums eines Reinigungs- und Desinfektionsgerätes vor dem Spülraum des Reinigungs- und Desinfektionsgerätes positionierbar ist, so dass in dieser Position die Tragschienen in ihrer Längsrichtung in Richtung zur Beschickungsrichtung verlaufen, **dadurch**
**gekennzeichnet, dass die** in dieser Position des Transportwagens dem Reinigungs- und Desinfektionsgerät abgewandte Seite des Traggestells (8) offen ausgebildet ist, wobei sich an dieser Seite des Traggestells (8) sich keine quer zu den Tragschienen (10, 11) erstreckende Verbindungsstrebe befindet,
so dass ein Verwender beim Einschieben eines vom Transportwagen aufgenommenen Spülkorbs (5) in das Reinigungs- und Desinfektionsgerät zwischen die Tragschienen (10, 11) des Transportwagens treten und auf das Reinigungs- und Desinfektionsgerät zugehen kann.

2. Transportwagen nach Anspruch 1, **dadurch gekennzeichnet, dass** das Traggestell (8) anderendseitig der Tragschienen (10 ,11) eine sich quer zu den Tragschienen (10 ,11) erstreckende Verbindungsstrebe (20) aufweist.

3. Transportwagen nach Anspruch 2, **dadurch gekennzeichnet, dass** die Verbindungsstrebe (20) an die Tragschienen (10, 11) angeschlossen ist.

4. Transportwagen nach einem der vorhergehenden Ansprüche, **dadurch gekennzeichnet, dass** eine Tragschiene (10 ,11) eine Vielzahl von in Richtung der Tragschiene (10, 11) hintereinander angeordnete erste Laufrollen aufweist.

5. Transportwagen nach einem der vorhergehenden Ansprüche, **dadurch gekennzeichnet, dass** eine Tragschiene (10 ,11) eine Mehrzahl von in Richtung der Tragschiene (10, 11) hintereinander angeordnete zweite Laufrollen aufweist.

6. Transportwagen nach Anspruch 4 und 5, **dadurch gekennzeichnet, dass** die ersten Laufrollen die zweiten Laufrollen in Höhenrichtung (21) überragen.

7. Transportwagen nach einem der Ansprüche 4 bis 6, **dadurch gekennzeichnet, dass** die ersten Laufrollen als Korblaufrollen (19) der Aufnahme eines Spülkorbs (5) dienen.

8. Transportwagen nach einem der Ansprüche 5 oder 6, oder nach Anspruch 7 wenn dieser abhängig von einem der Ansprüche 5 oder 6, **dadurch gekennzeichnet, dass**
die zweiten Laufrollen als Wannenlaufrollen (18) der Aufnahme einer unterhalb des Spülkorbs (5) angeordneten Wanne (12) dienen.

9. Transportwagen nach Anspruch 8, **dadurch gekennzeichnet, dass** die Wanne (12) einendseitig einen Korbmitnehmer (16) aufweist.

10. Transportwagen nach einem der vorhergehenden Ansprüche, **dadurch gekennzeichnet, dass** das Traggestell (8) auf seiner der offenen Seite (15) gegenüberliegenden Seite einen mit dem Reinigungs- und Desinfektionsgerät zusammenwirkenden Verriegelungsmechanismus (22) aufweist.

## Claims

1. Transport trolley for rinsing baskets of a cleaning and disinfection device, comprising a support frame (8) which has two support rails (10, 11) spaced apart from one another for receiving a rinsing basket (5), it being possible, for the purpose of loading a washing chamber of a cleaning and disinfection device, to position the transport trolley in front of the washing chamber of the cleaning and disinfection device so that, in this position, the support rails extend in their longitudinal direction in the direction of the loading direction, **characterised in that** the side of the support frame (8) that faces away from the cleaning and disinfection device in this position of the transport trolley is open, with no connecting strut that extends transversely to the support rails (10, 11) being located on this side of the support frame (8) so that a user, when inserting a rinsing basket (5) received by the transport trolley into the cleaning and disinfection device, can step between the support rails (10, 11) of the transport trolley and approach the cleaning and disinfection device.

2. Transport trolley according to claim 1, **characterised in that** the support frame (8), on the other end of the support rails (10, 11), has a connecting strut (20) extending transversely to the support rails (10, 11).

3. Transport trolley according to claim 2, **characterised in that** the connecting strut (20) is connected to the support rails (10, 11).

4. Transport trolley according to any of the preceding claims, **characterised in that** one support rail (10, 11) has a plurality of first rollers arranged one behind the other in the direction of the support rail (10, 11).

5. Transport trolley according to any of the preceding claims, **characterised in that** one support rail (10, 11) has a plurality of second rollers arranged one behind the other in the direction of the support rail (10, 11).

6. Transport trolley according to claim 4 and claim 5, **characterised in that** the first rollers project beyond the second rollers in the vertical direction (21).

7. Transport trolley according to any of claims 4 to 6, **characterised in that** the first rollers are used as basket rollers (19) to accommodate a rinsing basket (5).

8. Transport trolley according to either claim 5 or claim 6, or according to claim 7 when dependent on either claim 5 or claim 6, **characterised in that** the second rollers are used as tub rollers (18) to accommodate a tub (12) arranged below the rinsing basket (5).

9. Transport trolley according to claim 8, **characterised in that** the tub (12) has a basket driver (16) on one end.

10. Transport trolley according to any of the preceding claims, **characterised in that** the support frame (8) has, on its side opposite the open side (15), a locking mechanism (22) that interacts with the cleaning and disinfection device.

## Revendications

1. Chariot de transport pour paniers de lavage d'un appareil de nettoyage et de désinfection, comportant un cadre de support (8), lequel présente deux rails de support (10, 11) espacés l'un de l'autre et destinés à recevoir un panier de lavage (5), le chariot de transport pouvant être positionné devant l'espace de lavage de l'appareil de nettoyage et de désinfection dans le but de charger un espace de lavage d'un appareil de nettoyage et de désinfection, de telle sorte que, dans cette position, les rails de support s'étendent dans leur direction longitudinale en direction de la direction de chargement, **caractérisé en ce que**, dans cette position du chariot de transport, le côté du cadre de support (8) opposé à l'appareil de nettoyage et de désinfection est ouvert, une entretoise de liaison s'étendant transversalement aux rails de support (10, 11) ne se trouvant pas sur ledit côté du cadre de support (8), de telle sorte qu'un utilisateur peut insérer un panier de lavage (5) reçu par le chariot de transport dans l'appareil de nettoyage et de désinfection entre les rails de support (10, 11) et le rapprocher de l'appareil de nettoyage et de désinfection.

2. Chariot de transport selon la revendication 1, **caractérisé en ce que** le cadre de support (8) présente, du côté d'une autre extrémité des rails de support (10, 11), une entretoise de liaison (20) s'étendant transversalement aux rails de support (10, 11).

3. Chariot de transport selon la revendication 2, **caractérisé en ce que** l'entretoise de liaison (20) est raccordée aux rails de support (10, 11).

4. Chariot de transport selon l'une des revendications précédentes, **caractérisé en ce qu'**un rail de support (10, 11) présente une pluralité de premiers galets de roulement disposés les uns derrière les autres en direction du rail de support (10, 11).

5. Chariot de transport selon l'une des revendications précédentes, **caractérisé en ce qu'**un rail de support (10, 11) présente une pluralité de seconds galets de roulement disposés les uns derrière les autres en direction du rail de support (10, 11).

6. Chariot de transport selon les revendications 4 et 5, **caractérisé en ce que** les premiers galets de roulement surpassent les seconds galets de roulement dans la direction verticale (21).

7. Chariot de transport selon l'une des revendications 4 à 6, **caractérisé en ce que** les premiers galets de roulement servent de galets de roulement de panier (19) pour recevoir un panier de lavage (5).

8. Chariot de transport selon l'une des revendications 5 ou 6, ou selon la revendication 7 si celle-ci dépend de l'une des revendications 5 ou 6, **caractérisé en ce que** les seconds galets de roulement servent de galets de roulement d'auge (18) pour recevoir une auge (12) disposée en dessous du panier de lavage (5).

9. Chariot de transport selon la revendication 8, **caractérisé en ce que** l'auge (12) présente un entraîneur de panier (16) du côté d'une extrémité.

10. Chariot de transport selon l'une des revendications précédentes, **caractérisé en ce que** le cadre de support (8) présente un mécanisme de verrouillage (22) coopérant avec l'appareil de nettoyage et de désinfection sur son côté opposé au côté ouvert (15).
